# EUROPEAN PATENT SPECIFICATION

(11) **EP 3 367 978 B1**
(45) Date of publication and mention of the grant of the patent: **08.01.2020**
(21) Application number: 16838064.0
(22) Date of filing: 28.10.2016
(51) Int. Cl.: A61F 2/95, A61F 2/962, A61B 90/70

(54) **SYSTEMS AND METHODS FOR REMOVING AIR FROM STENT-GRAFTS AND OTHER MEDICAL DEVICES**
SYSTEME UND VERFAHREN ZUR ENTFERNUNG VON LUFT AUS STENTIMPLANTATEN UND ANDEREN MEDIZINISCHEN VORRICHTUNGEN
SYSTÈMES ET PROCÉDÉS POUR ÉLIMINER L'AIR D'ENDOPROTHÈSES ET D'AUTRES DISPOSITIFS MÉDICAUX

(30) Priority: 28.10.2015 US 201562247287 P
(43) Date of publication of application: 05.09.2018
(73) Proprietor: Mokita Medical GmbH, 13086 Berlin (DE)
(72) Inventor: KOLBEL, Tilo, 20251 Hamburg (DE)
(74) Representative: Gill Jennings & Every LLP
(86) International application number: PCT/IB2016/001908
(87) International publication number: WO 2017/072592

(56) References cited:
- EP-A2- 1 779 818
- WO-A1-2006/089517
- WO-A2-02/054990
- WO-A2-03/002020
- US-A1- 2014 277 403

## Description

### FIELD OF THE INVENTION

The present invention relates to devices, systems, and methods for removing gases from stent-grafts and their delivery systems , to reduce the risk of air embolism.

### BACKGROUND

Endovascular aortic repair (EVAR) is a type of endovascular surgery used to treat pathology of the aorta. The most common EVAR treatment is of an abdominal aortic aneurysm, but many different types of aortic pathologies are treated by EVAR. When used to treat thoracic aortic disease, the procedure is then specifically termed TEVAR (thoracic endovascular aortic/aneurysm repair). The procedure involves placement of an expandable stent-graft within the aorta to treat the aortic disease without operating directly on the aorta. In 2003, EVAR surpassed open aortic surgery as the most common technique for repair of abdominal aortic aneurysm, and in 2010, EVAR accounted for 78% of all intact abdominal aortic aneurysm repair in the United States.

The procedure is carried out in a sterile environment under x-ray fluoroscopic guidance by a vascular surgeon, cardiac surgeon, interventional radiologist, general surgeon, or interventional cardiologist. The patient's femoral arteries are generally accessed percutaneously, e.g., with a surgical incision or direct puncture in the groin. Vascular sheaths are introduced into the patient's femoral arteries, through which one or more guide wires, catheters, and the stent-graft are introduced. The stent-graft acts as an artificial lumen for blood to flow through, thereby substantially isolating the aneurysm sac from direct blood flow and blood-pressure and thereby preventing further enlargement and rupture. The stent-graft is compressed into a catheter, introducer sheath, or other delivery system that allows the compressed stent-graft to be introduced from the femoral arteries to the intended place of deployment.

A stent-graft is typically an assembly of a fabric material and a metal frame or metal springs/stents and mounted on a catheter assembly. When introduced into the vasculature, stent-grafts are constrained to a smaller diameter to enable introduction by different techniques, such as a constraining sleeve or by loading into an introducer sheath. Stent-grafts, stents, and their catheter assemblies are typically produced, constrained, packed and, sterilized under room-air conditions. Consequently, spaces within a constraining sleeve or sheath that are not filled by the stent-graft or stent and/or the catheter assembly generally contain room air. For sterilization, the assemblies are packed in packaging, which is permeable for gas and are sterilized, e.g., using vacuum with ethyleneoxide-containing gas. The gas is removed by repeated vacuum and room air ventilation as a later step of the gassterilization process. Thus, when the product is delivered in its sterile packaging there is generally air present within the stent-graft assembly.

In the operating theatre, the stent-graft assemblies are unpacked from their packaging under sterile conditions. Air is partially removed from some stent-grafts and their catheter assemblies prior to introduction into the vasculature typically by flushing the sheath with isotonic solutions such as saline through flushing ports that are part of the catheter assemblies. Stent-grafts that are constrained using a sleeve, such as the Gore TAG and cTAG device, are typically introduced into the vasculature without flushing to remove the room-air from the assembly.

It is well recognized that deployment of stent-grafts in the thoracic aorta involves a significant risk for stroke. It has been reported to be as high as 10% and is a major drawback of TEVAR.

While retrospective studies have been done, the pathomechanism of stroke as a complication of TEVAR is not well known. Generally, the main source for strokes are thought to be embolism by particles from thrombotic and atherosclerotic material adherent to the aortic wall, which is released by manipulation during deployment by wires, catheters, sheaths and the stent graft. Air-embolism by release of trapped air from the stent-graft during TEVAR may be a significant source of such strokes despite flushing techniques; however, it has been difficult to detect such events since the trapped air is not visible on fluoroscopy and they may only first recognized after the patient has woken up.

The risk of air-embolism and stroke during open surgery is well known and preventive strategies have been employed, e.g., in open cardiac surgery and neuro-surgery. Preventive strategies to avoid the introduction of air within endovascular devices into the human body include extensive saline flushing to mechanically squeeze out the air, which is present in catheters, stents (uncovered metal stents), coils, and other devices prior to introduction of these devices into the patient's vasculature. Such flushing with saline generally works well in these applications as air may be removed almost completely and so such flushing is generally part of the instructions for use of these devices. To what extend air is actually removed from such devices and how much air remains and is introduced into the vasculature is not well studied.

With stent-grafts (prosthetic vascular grafts supported by metal stents), flushing with saline solution may not work well to remove air prior to introduction into the body. However, it is the method that is widely recommended and used today in most procedures. Because stent-grafts are combinations of stents with a fabric-covering, traditional mechanical flushing with saline may not work well because the fabric significantly hampers the ability to completely drive out the air. Also, factors like the degree of compression may influence the amount of "trapped air."

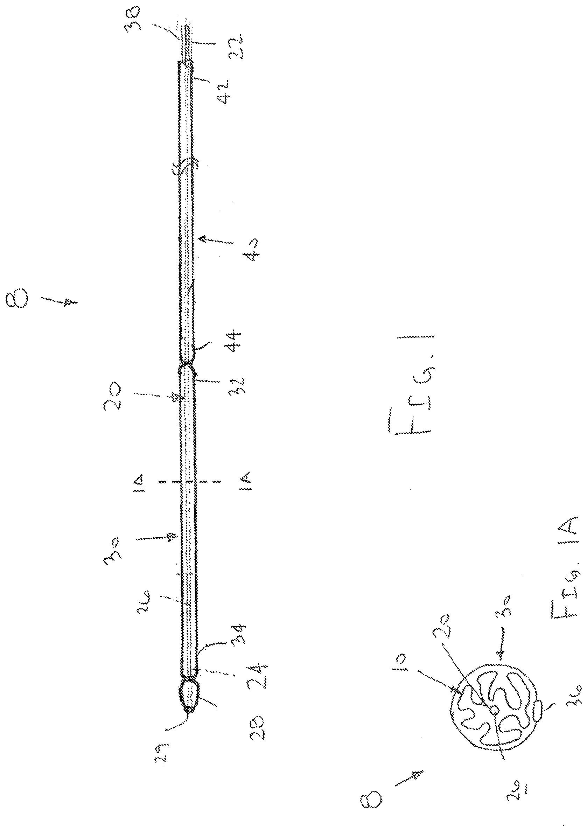

Another factor is the presence of side-branches and other advanced tools in modern stent-grafts and their delivery-systems, which may create pockets where air may be compressed during flushing, but not squeezed out. The trapped air may then be released during intravascular deployment of the procedure but may not be visually recognized during the procedure since air is not visible under fluoroscopy, which is generally used for such procedures. The released air may become visible on postoperative CT-scans after EVAR for abdominal aortic aneurysms in the aneurysm-sac days after the procedure, e.g., as shown in FIG. 1. Such occurrences are largely ignored because this air does not seem to cause much harm and is expected to be resorbed within weeks. The amount of air present in tubular sheath-constrained stent-grafts after flushing with saline according to the instructions for use has recently been published. See Rohlffs F, Tsilimparis N, Saleptsis V, Diener H, Debus ES, Kolbel T. Air Embolism During TEVAR: Carbon Dioxide Flushing Decreases the Amount of Gas Released From Thoracic Stent-Grafts During Deployment. J Endovasc Ther*.* Epub ahead of print October 26, 2016. DOI: 10.1177/1526602816675621.

Trapped air may also be released when stent-grafts are deployed in segments of the aorta, which are close to brain-supplying arteries, the aortic trunk vessels, e.g., the innominate artery, left common carotid artery, and left subclavian artery. When such trapped air is released, there is a risk of air embolization into the brain. The same is true if these stent-grafts are released close to the coronary arteries, giving rise to a risk for air-embolization into the coronary arteries with a risk for myocardial infarction. Thus, insufficient removal of air from stent-grafts and/or their delivery systems before they are introduced into the vasculature may be a significant source of stroke during TEVAR.

Air is also known to be released from other medical devices used in neuroradiological procedures. For example, stents and coils and their delivery-assemblies, which are introduced in the arteries of the brain, may also contain air, which may potentially cause damage in the brain.

Accordingly, devices and methods that facilitate removing air or other gases from medical devices, particularly stent-grafts, stents, coils and their delivery systems, to reduce the risk of embolism would be useful.

WO 02/054990 A2 discloses a process comprising a step of rinsing commercially available sterile stents with high pressure immediately before implantation for eliminating residual surface stent contaminants and reducing inflammation elicited by stent struts.

### SUMMARY

The present invention is directed to devices and methods for removing gases from stent-grafts and their delivery systems, to reduce the risk of air embolism as disclosed in the appended claims. More particularly, the present invention is directed to systems and methods for flushing medical devices and/or for loading such devices into delivery systems without substantial exposure to air once flushed.

For example, the systems and methods herein may involve "de-airing" a sleeve-constrained stent-graft before the stent-graft is introduced into the human vasculature. De-airing is principally done by replacing trapped air by other gases or fluids, which are better tolerated and have a decreased risk of embolization. Typical flushing gases may include one or more of carbon dioxide, oxygen, argon, helium or other gases, which are better tolerated within the vasculature. Flushing liquids include one or more of degassed or partially degassed solutions or chemicals with a high solubility of respiratory gases, such as perfluorochemicals.

In accordance with one embodiment, a flushing device is provided that includes an elongate tubular member including a first end, a second end, and a chamber therein extending between the first and second ends; first and second ports spaced apart from one another along the tubular member and communicating with the chamber; and one or more sources of flushing fluid connectable to one or both of the first and second ports to create a flushing circuit delivering flushing fluid into the first port, through the chamber, and out the second port to remove air or other gases from the chamber.

In accordance with another embodiment, a flushing device is provided that includes an elongate tubular member including a first end, a second end, a central chamber therein extending between the first and second ends; first and second ports spaced apart from one another along the tubular member; first and second annular chambers at least partially surrounding the central chamber, the first annular chamber communicating with the first port and including a first opening communicating with the central chamber at the first end of the tubular member, the second annular chamber communicating with the second port and including a second opening communicating with the central chamber at the second end of the tubular; and one or more sources of flushing fluid connectable to one or both of the first and second ports to create a flushing circuit delivering flushing fluid into the first port, through the chamber, and out the second port to remove air or other gases from the chamber.

In accordance with yet another embodiment, a system is provided for flushing a medical device before introduction into a patient's body that includes an elongate tubular member including a first end, a second end, and a chamber therein extending between the first and second ends; an introducing assembly carrying a stent-graft received within the chamber; first and second ports spaced apart from one another along the tubular member and communicating with the chamber; and one or more sources of flushing fluid connectable to one or both of the first and second ports to create a flushing circuit delivering flushing fluid into the first port, through the chamber, and out the second port to remove air or other gases from the stent-graft.

In accordance with still another embodiment, a system is provided for flushing a medical device before introduction into a patient's body that includes an elongate tubular member including a first end, a second end, a central chamber therein extending between the first and second ends; an introducing assembly carrying a stent-graft received within the chamber; first and second ports spaced apart from one another along the tubular member; first and second annular chambers at least partially surrounding the central chamber, the first annular chamber communicating with the first port and including a first opening communicating with the central chamber at the first end of the tubular member, the second annular chamber communicating with the second port and including a second opening communicating with the central chamber at the second end of the tubular member; and one or more sources of flushing fluid connectable to one or both of the first and second ports to create a flushing circuit delivering flushing fluid into the first port, through the chamber, and out the second port to remove air or other gases from the chamber.

In accordance with yet another embodiment, a system is provided for flushing a medical device before introduction into a patient's body that includes an elongate tubular member including a first end, a second end, a chamber therein extending between the first and second ends, and first and second ports spaced apart from one another along the tubular member and communicating with the chamber; an introducing assembly carrying a stent-graft received within the chamber; and a flushing machine including one or more reservoirs and a circuit communicating with the first and second ports for selectively introducing one or more flushing fluids into the chamber to flush the introducing assembly and stent-graft and removing gases and excess flushing fluid from the chamber.

In accordance with another embodiment, a method is provided for removing gas from a stent-graft that includes providing a flushing device including a chamber extending between first and second ends thereof; introducing an introducing assembly carrying the stent-graft constrained within a sleeve into the chamber; and flushing the chamber with one or more flushing fluids.

Other aspects and features of the present invention will become apparent from consideration of the following description taken in conjunction with the accompanying drawings.

### BRIEF DESCRIPTION OF THE DRAWINGS

The drawings illustrate exemplary embodiments of the invention, in which:
FIG. 1 is a side view of an introducing assembly including a sleeve-constrained stent-graft and a pusher member carried on a central cannula.
FIG. 1A is a cross-section of the introducing assembly of FIG. 1 taken across 1A-1A.
FIG. 2 is a cross-sectional view of an exemplary embodiment of a flushing device including a tubular body with inlet and outlet ports for flushing and/or loading an introducing assembly and/or stent-graft.
FIG. 3 is a cross-sectional view of the flushing device of FIG. 2 with the introducer device and stent-graft of FIG. 1 received therein.
FIG. 4 is a cross-sectional view of another exemplary embodiment of a flushing device including a tubular body with inlet and outlet ports for flushing and/or loading an introducing assembly and/or stent-graft.
FIGS. 4A and 4B are cross-sectional details of the flushing device of FIG. 4 taken at locations 4A-4A and 4B-4B, respectively.
FIG. 5 is a cross-sectional view of the flushing device of FIG. 4 with a stent-graft carried by an introducing assembly received therein.
FIGS. 6A and 6B are details showing a delivery device being connected to a flushing device to transfer an introducing assembly from the flushing device into the delivery device after flushing.
FIG. 7 is a side view of an exemplary embodiment of a splittable sheath including a flushing port for flushing a stent-graft loaded therein.
FIGS. 7A and 7B are cross-sectional details of the sheath of FIG. 7 taken at locations 7A-7A and 7B-7B, respectively.

### DETAILED DESCRIPTION OF THE EXEMPLARY EMBODIMENTS

Reducing the amount of air present in a stent-graft, stent, or other prosthesis and their delivery systems may reduce the incidents of stroke and/or other damage that may result from air embolism. In accordance with an exemplary embodiment, systems and methods are provided that including using a flushing device to flush medical devices, such as stent-grafts and/or their delivery systems.

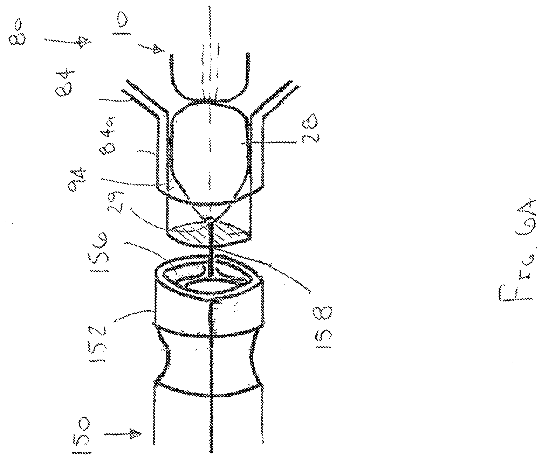

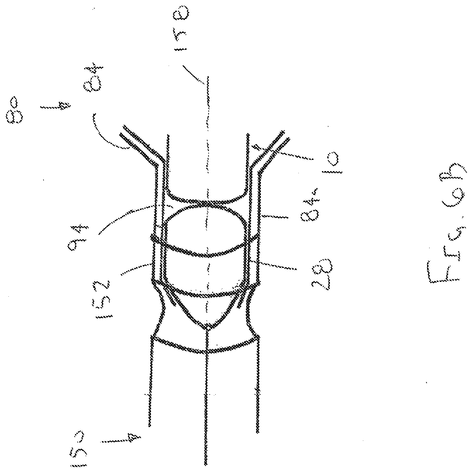

Turning to the drawings, FIG. 1 shows an exemplary embodiment of a stent-graft 10 carried by an introducing assembly 8, which may be flushed using the systems and methods described herein. Generally, the introducing assembly 8 includes an elongate central cannula or first shaft 20, a sleeve or cover 30, and a pusher or second shaft 40 for delivering the stent-graft 10, e.g., via an introducer sheath, catheter, or other delivery device (not shown, see, e.g., sheath 150 shown in FIGS. 6A and 6B) and/or a guidewire or other rail (also not shown), as described elsewhere herein. The cannula 20 generally is an elongate tubular member including a proximal end 22, a distal end 24 sized for introduction into a patient's body, and one or more lumens extending therebetween, e.g., a lumen 26 sized for receiving a guidewire or other rail (not shown). An enlarged distal tip 28 may be provided on the distal end 24, e.g., having a tapered, rounded, and/or other atraumatic shape to facilitate introduction of the introducing assembly 8 into a delivery device and/or into a patient's body.

The stent-graft 10 is loaded on the cannula 20 adjacent the distal tip 28 in a compressed or contracted condition and a cover or sleeve 30 is provided to maintain the stent-graft 10 in the constrained condition, as best seen in FIG. 1A, and/or provide a substantially smooth transition from the distal tip 28. In an exemplary embodiment, the sleeve 30 may be a sheet of material including proximal and distal ends 32, 34, which is wrapped around the stent-graft 10 and has its opposite edges secured together, e.g., using a running suture or other filament 36 that extends between the proximal and distal ends 32, 34. The filament 36 may then extend proximally to the proximal end 22 of the cannula 20 such that a proximal end 38 of the filament 36 may be pulled to release the sleeve 30 during deployment of the stent-graft 10, as described elsewhere herein. In one embodiment, the filament 36 may pass through a dedicated lumen (not shown) in the cannula 20, e.g., adjacent the central lumen 26 or, alternatively, may pass through the central lumen 26. In a further alternative, the filament 36 may pass through the pusher member 30, e.g., adjacent the cannula 20 or within a dedicated lumen (not shown) of the pusher member 30.

Alternatively, the sleeve 30 may include one or more weakened regions (not shown) along which the filament 36 may extend such that the proximal end 38 of the filament 36 may be pulled to tear the weakened region(s) to release the sleeve 30 and allow deployment of the stent-graft 10. In one embodiment, the sleeve 30 may be attached to the stent-graft 10 at one or more locations, e.g., along a longitudinal line, such that the sleeve 30 remains attached to and/or partially around the stent-graft 10 but opens as the stent-graft 10 expands. Alternatively, the sleeve 30 may be separable from the stent-graft 10, and may remain coupled to the filament 36, which may be used to remove the released sleeve 30.

The pusher or stopper member 40 is an elongate tubular member including a proximal end 42 and a distal end 44 positioned adjacent the stent-graft 10 and sleeve 30. The pusher member 40 may include a lumen that receives the cannula 20, e.g., such that the pusher member 40 and cannula 20 are movable axially relative to one another. Alternatively, the pusher member 40 and cannula 20 may be axially fixed relative to one another or, in a further alternative, a fixed stop (not shown) may be attached to the cannula 20 adjacent the stent-graft 10 instead of the pusher member 40.

Optionally, a handle or hub (not shown) may be provided on the proximal end 22 of the cannula 20 including a port (also not shown) communicating with the lumen 26, e.g., including one or more seals, valves, and/or connectors, e.g., a female Luer lock fitting, to allow a source of fluid to be coupled to the port, e.g., for flushing the lumen 26, and/or to accommodate receiving a guidewire or other instrument (not shown) through the lumen 26. The hub may include one or more actuators or features, as desired for deploying the stent-graft 10. For example, the hub may include a side port (not shown) and the proximal end 38 of the filament 36 may pass through the side port such that a user may pull the proximal end 38 to release the sleeve 30. Alternatively, the proximal end 38 may be coupled to an actuator on the hub such that the actuator may be manipulated to pull the filament 36 and open the sleeve 30.

For example, during use, the introducing assembly 8 (carrying the stent-graft 10 constrained by the sleeve 30) may be introduced into a patient's body, e.g., from a percutaneous entry site, and advanced to a target location, e.g., within the patient's aorta, which is the site of an aneurysm (not shown). In an exemplary method, a guidewire may be introduced from the entry site and manipulated to position the guidewire adjacent the target location. An introducer sheath may be advanced over the guidewire and also positioned at the target location, whereupon the guidewire may be backloaded through the lumen 26 of the cannula 20, e.g., via opening 29 in the distal tip 28, and then the introducing assembly 8 may be advanced over the guidewire through the introducer sheath. Once properly positioned, the distal end 24 of the cannula 20 carrying the sleeve 30 and the stent-graft 10 may be exposed from the introducer sheath at the target site, and then the sleeve 30 opened to expose the stent-graft 10, e.g., by pulling the filament 36.

The stent-graft 10 may be configured to resiliently expand within the target location automatically upon being exposed. Alternatively, the introducing assembly 8 may include a balloon or other expandable member (not shown) under the stent-graft 10, which may be inflated or otherwise manipulated to expand the stent-graft 10. In one embodiment, the sleeve 30 may remain at the target delivery site, e.g., captured between the stent-graft 10 and the surrounding vessel wall or other tissue. Alternatively, the sleeve 30 may be removed from around the stent-graft 10, e.g., withdrawn into the introducer sheath using the filament 36 or other feature (not shown).

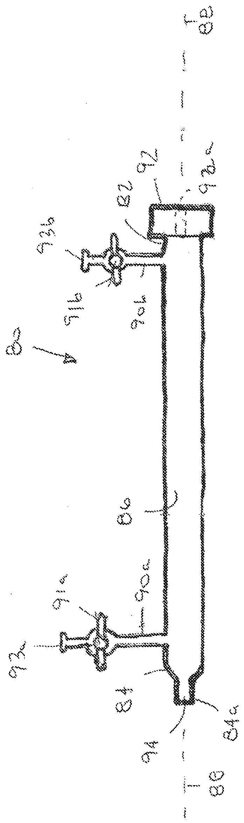

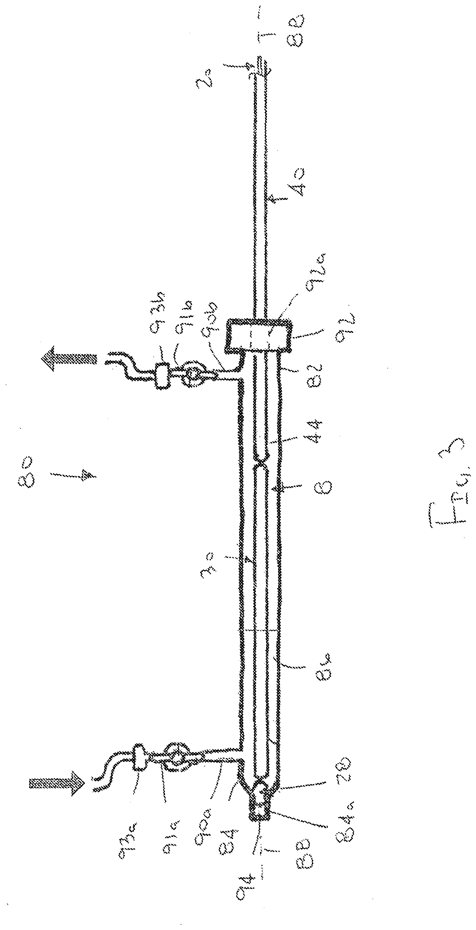

Prior to introduction of the introducing assembly 8 and stent-graft 10 into the patient's body (e.g., via an introducer sheath), the systems and methods herein may be used to flush the stent-graft 10, e.g., to remove air or other gases. For example, turning to FIGS. 2 and 3, an exemplary embodiment of a flushing and/or loading device or tube 80 is shown that includes a first or proximal end 82, a second or distal end 84 opposite the first end 82, and a flushing chamber 86 extending between the first and second ends 82, 84 along longitudinal axis 88. The chamber 86 may have a substantially circular cross-section along its length, e.g., having a diameter larger than the outer diameter of the introducing assembly 8. Alternatively, the chamber 86 may have other cross-sections, e.g., including a groove or pocket (not shown) extending at least partially along a side wall of the chamber 86 between the first and second ends 82, 84, which may collect gases that escape from the introducing assembly 8 and/or stent-graft 10 during flushing. Optionally, a separate port (also not shown) may be provided in the flushing device 80 that communicates with the groove or pocket to remove such escaped gases.

The first end 82 may include a hub 92, which may be removably coupled to the first end 82, e.g., by one or more of an interference fit, cooperating connectors, adhesive, and the like, to provide a fluid-tight seal when coupled to the first end 82. For example, the hub 92 may be removed to load an introducing assembly 8 carrying a stent-graft 10 or other medical device into the chamber 86, whereupon the hub 92 may be reconnected to the first end 82 over the pusher member 40 to seal the chamber 86 with the stent-graft 10 therein, as shown in FIG. 3.

The hub 92 may include one or more passages therethrough, e.g., passage 92a shown in FIG. 2 aligned along the axis 88, for receiving the pusher member 40 of the introducing assembly 8 therethrough, as shown in FIG. 3. For example, the passage 92a may include one or more valves, e.g., a hemostatic valve (not shown), which may provide a substantially fluid-tight seal, while accommodating insertion of the pusher member 40 into and/or axial movement of the pusher member 40 along the chamber 86, as described elsewhere herein.

Alternatively, the hub 92 may be substantially permanently attached to the first end 82 and the passage 92a may be sized and/or otherwise configured to accommodate introducing the introducing assembly 8 and stent-graft 10 into the chamber 86. For example, as shown in FIG. 3, the distal tip 28 followed by the sleeve 30 and pusher member 40 may be directed through the passage 92a into the chamber 86, e.g., by manipulating the pusher member 40 or a handle of the introducing assembly 8.

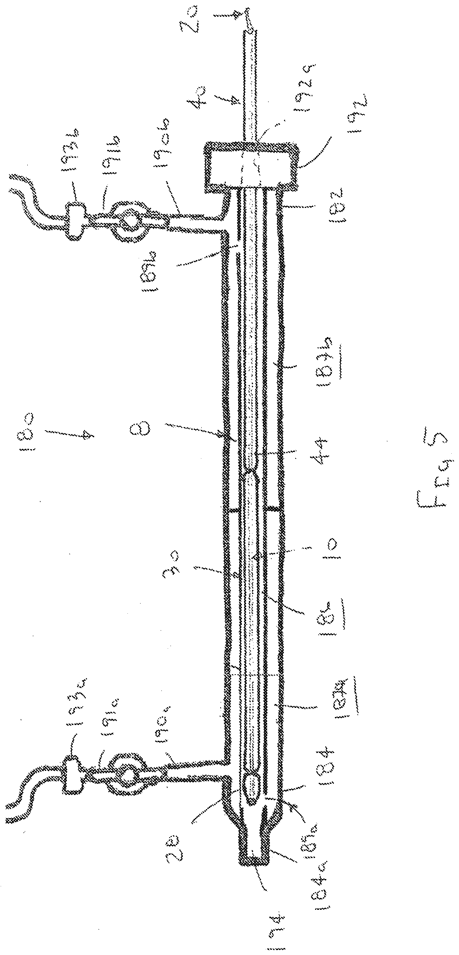

The second end 84 of the flushing device 80 may include an opening 94, which may be selectively opened and closed, e.g., to introduce the introducing assembly 8 and stent-graft 10, after flushing, into a delivery device (not shown), such as an introducer sheath 150, as shown in FIGS. 5A and 5B. For example, as shown, the second end 84 may taper to a nipple 84a including the opening 86 therein, which may be coupled with a hub of the delivery device. In one embodiment, the nipple 84a may include one or more connectors, e.g., a Luer lock fitting and the like (not shown), which may be coupled to corresponding connector(s) on the hub of the delivery device. Alternatively, the nipple 84a may be sized to slide into a port on the delivery device hub, e.g., through any seals, to allow the stent-graft 10 to be loaded through the opening 86 into a lumen of the delivery device without interference from the seal(s). Once the nipple 84a is coupled to or received in the delivery device, the introducing assembly 8 may be advanced to introduce the stent-graft 10 into the delivery device lumen and, optionally, to a distal end of the delivery device already introduced into a patient's body, as described elsewhere herein.

Optionally, the second end 84 may include one or more seals to provide a fluid-tight seal, e.g., to prevent gases or other material passing through the opening 94 into the chamber 86. For example, the one or more seals may prevent gases or other materials from entering the chamber 86 while accommodating transfer of the introducing assembly 8 through the opening 94 into a delivery device. In an alternative embodiment, a removable cap or other seal (not shown) may be removably coupled to the nipple 84a to selectively open and close the opening 94. In another alternative, a stopcock or a sliding valve (also not shown) may be provided at the second end 84, which may be movable between open and closed positions to open and close the opening 94. In yet another alternative, a fluid-tight membrane (also not shown) may cover the opening 94, which may be penetrated or torn (e.g., including one or more perforations or weakened regions that fail upon encountering a threshold force, e.g., when the distal tip 28 is advanced into the opening 94 into contact with the membrane), e.g., when the introducing assembly 8 is directed through the opening 94 into a delivery device (not shown).

In yet another alternative, as shown in FIG. 3, the distal tip 28 may be sized to sealingly and/or slidably engage the opening 94, e.g., having sufficient flexibility to at least partially enter the opening 94 while providing a fluid-tight seal to prevent gases or material passing through the opening 94. In this alternative, the distal tip 28 may be sized and/or sufficiently flexible such that the distal tip 28 may be directed through the opening 94, e.g., when transferring the flushed introducing assembly 8 into a delivery device.

With continued reference to FIGS. 2 and 3, the flushing device 80 also includes one or more ports 90 for introducing flushing fluids into, applying a vacuum to the chamber 86, and/or collecting fluids flushed through the chamber 86. The ports 90 may be formed as rigid nipples and/or flexible tubing including one or more valves 91 for selectively opening and closing the ports 90 and one or more connectors, e.g., Luer lock fittings 93, for coupling the ports 90 to a source of flushing fluid and/or vacuum.

For example, as shown in FIG. 3, a first port 90a, e.g., immediately adjacent the second end 84 of the flushing device 80, may be coupled to tubing communicating with a source of flushing fluid, e.g., a syringe, pump, or other container (not shown) including one or more gases, PFC solutions, saline, and the like, as described elsewhere herein. A second port 90b, e.g., immediately adjacent the first end 82 of the flushing device 80, may be coupled to tubing communicating with a collection source, e.g., a syringe, vacuum line, pump, collection chamber or container, and the like (also not shown).

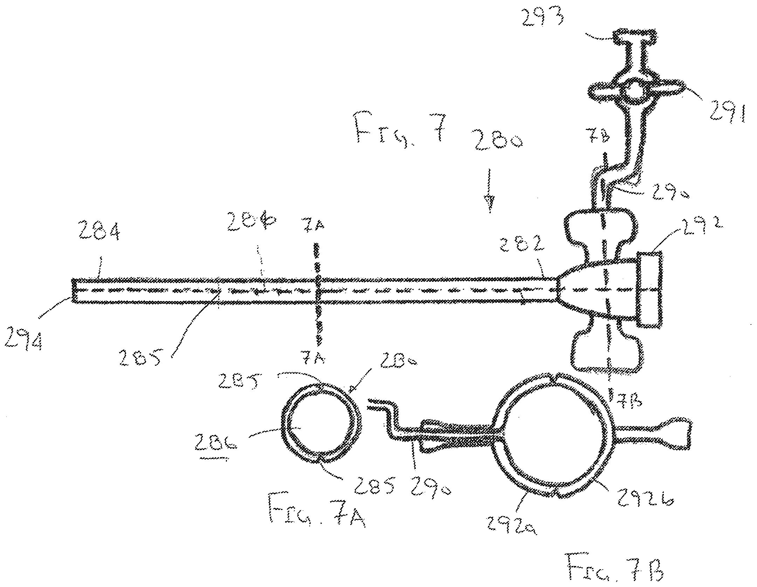

The flushing device tube 80 and hub 92 may be formed from substantially rigid material, e.g., glass, metal, plastic, or composite materials, e.g., that are gas-impermeable to prevent air or other external gases from passing into the chamber 86. Alternatively, the tube 80 may be formed from flexible, gas-impermeable material, e.g., to provide a flexible sleeve into which the stent-graft 10 may be loaded, flushed, and then transferred. For example, FIG. 7 shows an exemplary embodiment of a flexible flushing device 280 that may be used instead of the flushing device 80 shown in FIGS. 2 and 3. Optionally, such a flexible flushing device 280 may include one or more weakened regions 285, e.g., extending axially or otherwise between first and second ends 282, 284 thereof, which may facilitate splitting the flushing device 280, e.g., to remove the flushing device 280 from around the introducing assembly 8 and/or other devices after flushing, as described elsewhere herein.

Returning to FIGS. 2 and 3, a method will now be described for flushing a medical device, such as an introducing assembly 8 carrying a stent-graft 10, using the flushing device 80. For example, in one embodiment, the introducing assembly 8 may be loaded into the chamber 86 via the first end 82, e.g., by removing the hub 92 and introducing the distal tip 28 followed by sleeve 30 covering the stent-graft 10 into the first end 82. Once the stent-graft 10 is fully received within the chamber 86, the hub 92 may be attached or otherwise secured to the first end 82, e.g., by engaging one or more connectors, and the like.

Alternatively, the hub 92 may remain on the first end 82, and the introducing assembly 8 may be introduced through the passage 92a and positioned within the chamber 86. Optionally, if the chamber 86 is sealed before the introducing assembly 8 is introduced, the chamber 86 may be prefilled with a desired gas or flushing fluid, e.g., such that the gas or flushing fluid permeates into the sleeve 30 and/or stent-graft 10 upon introduction into the chamber 86, which may enhance flushing the introducing assembly 8 and/or stent-graft 10.

The opening 94 at the second end 84 may be sealed or closed, as described above, and the ports 90 may be closed such that the chamber 86 is isolated from the external environment of the flushing device 80. One or more sources of flushing fluid and/or vacuum may be coupled to the ports 90, whereupon the stopcocks 91 may be opened to flush the chamber 86, with the introducing assembly 8 and stent-graft 10 therein, one or more times. For example, a source of carbon dioxide or bio-inert gas may be coupled to the first port 90a and a source of vacuum may be coupled to the second port 90b to create a circuit that introduces the gas into the first port 90a, flushes the introducing assembly 8, particularly the stent-graft 10 within the sleeve 30 to replace air or other gases with the flush gas, and evacuates the air and excess flushing fluid out the second port 90b. Such flushing may continue for sufficient time to ensure that the flush gas substantially replaces any air within the chamber 86 and stent-graft 10.

Thereafter, a source of PFC solution may be coupled to the first port 90a and used to flush the gas. In addition or alternatively, a source of saline may be coupled to the first port 90a and used to flush the PFC solution and/or the previously introduced gas. It will be appreciated that any sequence of flushing fluids and/or procedures may be used, such as those described in the Rohlffs et al. publication identified above.

Alternatively, the port 90a may include multiple connectors (not shown) such that multiple sources may be connected at the same time, and the stopcock 91a may be selectively directed to different positions to allow a desired source to be delivered into the chamber 86. Thus, in this alternative, the stopcock 91a may be manually or mechanically switched between the different positions in any desired sequence to flush the chamber 86 and introducing assembly 8, e.g., corresponding to any of the methods described elsewhere herein.

In alternative embodiments, one or both of the ports 90 may be omitted and, instead, sources of flushing fluid and/or vacuum may be coupled to the opening 94 and/or passage 93 to provide a flushing circuit that may operate similarly to the methods just described. In addition or alternatively, a flushing machine (not shown) may be connected to the ports 90, which may be self-contained and/or may operate to introduce various flushing fluids and/or collect fluids once connected to the flushing device 80. For example, the machine may include multiple reservoirs containing different fluids, i.e., gases and/or liquids, that may be delivered into the chamber 86.

Once the introducing assembly 8 has been flushed, the introducing assembly 8 may be loaded into a delivery device, e.g., before or after introducing the delivery device into a patient's body. For example, FIGS. 6A and 6B are details showing a hub 152 of an introducer sheath 150 including a lumen or other passage 156 into which the introducing assembly 8 may be loaded after flushing. As shown, the hub 150 and the second end 84 of the flushing device 80 may be connected together, e.g., using one or more of an interference fit, mating connectors, and the like, thereby providing a fluid-tight seal between the hub 150 and the flushing device 80. The introducer assembly 8 may then be transferred through the opening 94 from the chamber 86 into the lumen 156 without exposing the introducer assembly 8 to the external environment, which may otherwise introduce air or other undesired materials into the introducing assembly 8.

For example, a guidewire or other rail 158 may already be positioned through the lumen 156 of the introducer sheath 150, e.g., used to introduce and/or guide the introducer sheath 150 through the patient's vasculature from a peripheral access site to a target location within the patient's body (not shown). The guidewire 158 may be backloaded through the opening 29 in the distal tip 28 and through the lumen 26 of the cannula 20 to facilitate guiding the introducing assembly 8 into the lumen 156. For example, the introducing assembly 8 may be advanced to slide the distal tip 28 through the opening 94, thereby maintaining a substantially fluid-tight seal to further prevent exposure of the introducing assembly 8 and stent-graft 10 to gases or the external environment. Once the distal tip 28, stent-graft 10, and the other components of the introducing assembly 8 enter the lumen 156 of the introducer sheath 150, the introducer assembly 8 may be advanced through the lumen 156, e.g., into a distal end of the introducer sheath 150, e.g., already positioned at the target location. The flushing device 80 may then be removed from the hub 150 and, optionally, torn or otherwise separated if the flushing device 80 includes weakened regions, similar to the flushing device 280 shown in FIG. 7, to remove the flushing device 80 entirely from the introducer sheath 150 and guidewire 158.

For example, as shown in FIG. 7, flushing and loading device 280 may include one or more weakened regions 285 extending between first and second ends 282, 284 thereof (e.g., shown in FIGS. 7 and 7A). After the introducing assembly 8 and stent-graft 10 have be transferred from the flushing device 280 into a delivery device (not shown), e.g., through opening 294, the flushing device 280 remains surrounding the pusher member 40 and/or other components of the introducing assembly 8 and/or guidewire. To remove the flushing device 280, the hub 292 may include two portions 292a, 292b including weakened regions, as shown in FIG. 7B, such that the two portions 292a, 292b may be pulled apart away from one another, causing the weakened region(s) 285 to fail and separate. This action causes the flushing device 280 to tear open or otherwise separate along the weakened regions 285 from the first end 282 towards the second end 284, e.g., until the flushing device 280 is separated into two pieces, which may then be discarded.

Once the introducing assembly 8 and stent-graft 10 are properly positioned at the target location, the hub 152 may be withdrawn proximally to expose and/or deploy the stent-graft 10, as described elsewhere herein.

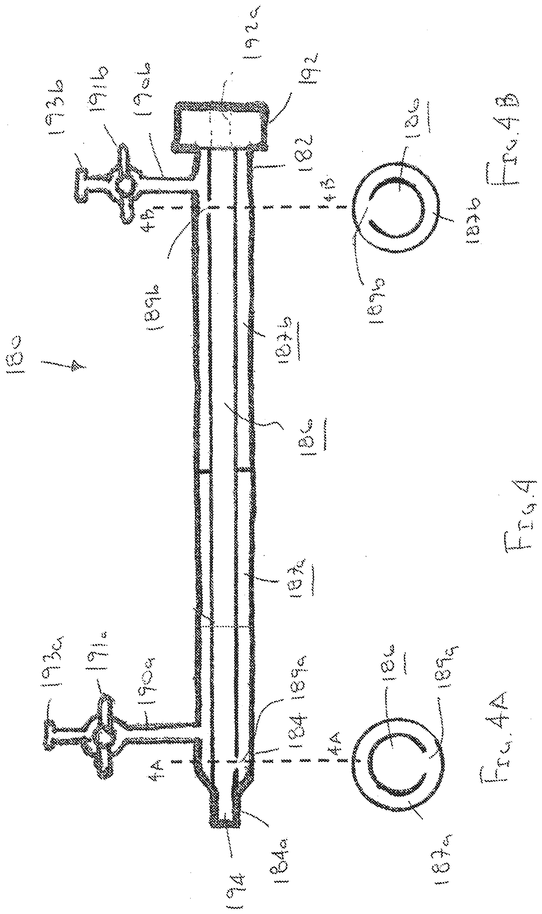

Turning to FIGS. 4 and 5, another example of a flushing and/or loading device 180 is shown. Similar to the other embodiments herein, the flushing device 180 generally includes first and second opposite ends 182, 184, a chamber 186 extending therebetween, a hub 192 on the first end 182 including a passage 192a, and an opening 194 in the second end 184. In addition, the flushing device 180 includes first and second ports 190 including stopcocks 191 and connectors 193, also generally similar to other embodiments herein.

However, unlike previous embodiments, the flushing device 180 includes annular chambers 187 surrounding the central chamber 186 (into which a stent-graft 10 and/or introducing device 110 is introduced). As can be seen, the ports 190 communicate directly with the annular chambers 187, and the annular chambers 187 communicate with the central chamber 186 via openings 189. The openings 189 are positioned at opposite ends of the flushing device 180, e.g., with a first opening 189a located immediate adjacent the end opening 194 and the second opening 189b located immediately adjacent the hub 192.

This configuration may facilitate introducing one or more flushing fluids to displace air or other gases within the chamber 186 and stent-graft 10 during flushing. For example, when a flushing fluid is introduced via the first port 190a, the fluid may initially fill the first annular chamber 187a and then enter the central chamber 186 via the first opening 189a. The flushing fluid will then be forced through the central chamber 186 along the flushing device 180 from the second end 184 towards the first 182, creating a desired pressure within the chamber 186 that may facilitate removing trapped gases within the stent-graft 10 and/or the introducing assembly 8. After reaching the first end 182, the fluid may exit the second opening 189b into the second annular chamber 187b, which may fill until the fluid is directed into the second port 190b, e.g., under vacuum from a source of vacuum or simply under the positive pressure within the chamber 186 into a container.

For example, as shown in FIG. 5, an introducing assembly 8 carrying a stent-graft 10 may be introduced into the chamber 186 of the flushing device 180 and flushed similar to other embodiments described herein. After the introducing assembly 8 and stent-graft 10 have been flushed in a desired manner, the introducing assembly 8 may be transferred to a delivery device, e.g., via the opening 194 in the second end 184, similar to previous embodiments.

In another alternative, the flushing chamber and one or more ports may be provided within a hub of a delivery device, rather than as a separate device. For example, in this alternative, one or more sources of flushing fluid and/or vacuum may be coupled to the port(s) of the hub to flush the chamber and stent-graft. Once flushing is completed, the stent-graft may be advanced from the chamber through a lumen of the delivery device, similar to other embodiments herein.

## Claims

1. A system for flushing a medical device before introduction into a patient's body, comprising:
an elongate tubular member including a first end, a second end, and a chamber therein extending between the first and second ends;
an introducing assembly carrying a stent-graft received within the chamber;
first and second ports spaced apart from one another along the tubular member and communicating with the chamber; and
one or more sources of perfluorocarbon solution connectable to one or both of the first and second ports to create a flushing circuit delivering perfluorocarbon solution into the first port, through the chamber, and out the second port to remove air or other gases from one or both of the stent-graft and the chamber.

2. The system of claim 1, wherein the chamber comprises a central chamber, and wherein the tubular member further comprises first and second annular chambers at least partially surrounding the central chamber, the first annular chamber communicating with the first port and including a first opening communicating with the central chamber at the first end of the tubular member, the second annular chamber communicating with the second port and including a second opening communicating with the central chamber at the second end of the tubular member.

3. The system of claim 1 or claim 2, further comprising a hub on the first end of the tubular member, the hub being removable from the first end to allow a medical device to be loaded into the chamber for flushing, the hub being reconnectable to the first end for sealing the chamber after loading the medical device into the chamber.

4. The system of claim 1 or claim 2, further comprising a hub on the first end of the tubular member including a passage therethrough for introducing a medical device into the chamber, the passage including one or more seals to accommodate introducing the medical device and provide a fluid-tight seal.

5. The system of any of any preceding claim, wherein the second end of the tubular member includes an opening for transferring a medical device within the chamber after flushing through the opening into a delivery device.

6. The system of claim 5, wherein the second end of the tubular member includes a mechanism for selectively closing the opening for sealing the chamber and opening the opening to allow transfer of the medical device through the opening.

7. The system of claim 5, further comprising a sealing member on the second end for sealing the opening, the sealing member being removable to allow the medical device to be transferred from the chamber through the opening.

8. The system of claim 5, further comprising a membrane on the second end providing a fluid-tight seal sealing the opening, the membrane including one or more weakened regions configured to tear or fail when subjected to a predetermined force.

9. The system of claim 5, further comprising a membrane on the second end providing a fluid-tight seal sealing the opening, the membrane configured to tear or fail when the medical device is being directed into the opening to allow the medical device to be transferred from the chamber into the delivery device.

10. The system of any preceding claim, wherein the tubular member comprises one or more weakened regions in a wall of the tubular member extending at least partially from the first end towards the second end, the tubular member comprising one or more elements for tearing the one or more weakened regions for opening or separating the tubular member to allow the tubular member to be removed from around an introducing assembly flushed in the chamber.

11. The system of claim 10, wherein the one or more elements comprise a hub at the first end including first and second portions that may be separated to cause the one or more weakened regions to tear from the first end towards the second end.

12. The system of any preceding claim, further comprising a first flushing fluid within the chamber such that a medical device introduced into the chamber is exposed to the first flushing fluid before introducing one or more additional flushing fluids into the chamber.

13. The system of any preceding claim, further comprising a source of saline connectable to the first port for flushing the lumen with the saline to remove the degassed or partially degassed solution or perfluorocarbon solution from one or both of the stent-graft and the chamber.

14. The system of any preceding claim, further comprising a source of flushing gas connectable to one of the first and second ports to create a circuit to replace air or other gases in one or both of the stent-graft and the chamber with the flushing gas prior to flushing with the perfluorocarbon solution, wherein the flushing gas is carbon dioxide or bio-inert gas.

15. A method for removing gas from a medical device, comprising:
providing a flushing device including a chamber extending between first and second ends thereof;
introducing an introducing assembly carrying a stent-graft constrained within a sleeve into the chamber; and
flushing the chamber with perfluorocarbon solution;
wherein the flushing device includes first and second ports at the first and second ends, respectively, and wherein flushing the chamber comprises:
coupling a source of perfluorocarbon solution to the first port;
coupling a collection source to the second port;
delivering perfluorocarbon solution from the source through the first port into the chamber; and
collecting flushed gas and excess perfluorocarbon solution from the chamber through the second port into the collection source.

## Patentansprüche

1. System zum Spülen einer medizinischen Vorrichtung vor der Einführung in den Körper eines Patienten, das Folgendes umfasst:
ein längliches röhrenförmiges Element, das Folgendes einschließt: ein erstes Ende, ein zweites Ende und eine Kammer darin, die sich zwischen dem ersten und zweiten Ende erstreckt;
eine Einführanordnung, die ein Stentgraft trägt, das innerhalb der Kammer aufgenommen ist;
ersten und zweiten Anschluss, die voneinander entlang des röhrenförmigen Elements beabstandet sind und mit der Kammer in Verbindung stehen; und
eine oder mehrere Quellen für Perfluorkohlenwasserstofflösung, die mit einem oder beiden des ersten und zweiten Anschlusses verbindbar sind, um einen Spülkreislauf zu erzeugen, der Perfluorkohlenwasserstofflösung in den ersten Anschluss, durch die Kammer und aus dem zweiten Anschluss abgibt, um Luft oder andere Gase aus einem oder beiden von dem Stentgraft und der Kammer zu entfernen.

2. System nach Anspruch 1, wobei die Kammer eine mittlere Kammer umfasst und wobei das röhrenförmige Element weiter eine erste und zweite Ringkammer umfasst, die zumindest teilweise die mittlere Kammer umgibt, wobei die erste Ringkammer mit dem ersten Anschluss in Verbindung steht und eine erste Öffnung einschließt, die mit der mittleren Kammer an dem ersten Ende des röhrenförmigen Elements in Verbindung steht, die zweite Ringkammer mit dem zweiten Anschluss in Verbindung steht und eine zweite Öffnung einschließt, die mit der mittleren Kammer an dem zweiten Ende des röhrenförmigen Elements in Verbindung steht.

3. System nach Anspruch 1 oder Anspruch 2, das weiter ein Ansatzstück an dem ersten Ende des röhrenförmigen Elements umfasst, wobei das Ansatzstück von dem ersten Ende entfernbar ist, um das Eingeben einer medizinischen Vorrichtung in die Kammer zum Spülen zu ermöglichen, wobei das Ansatzstück mit dem ersten Ende zum Abdichten der Kammer nach dem Eingeben der medizinischen Vorrichtung in die Kammer wieder verbindbar ist.

4. System nach Anspruch 1 oder Anspruch 2, das weiter ein Ansatzstück an dem ersten Ende des röhrenförmigen Elements umfasst, das einen Durchgang dahindurch für die Einführung einer medizinischen Vorrichtung in die Kammer einschließt, wobei der Durchgang eine oder mehrere Dichtungen einschließt, um die Einführung der medizinischen Vorrichtung anzupassen und eine flüssigkeitsdichte Abdichtung bereitzustellen.

5. System nach einem vorstehenden Anspruch, wobei das zweite Ende des röhrenförmigen Elements eine Öffnung für die Überführung einer medizinischen Vorrichtung innerhalb der Kammer nach dem Spülen durch die Öffnung in eine Abgabevorrichtung einschließt.

6. System nach Anspruch 5, wobei das zweite Ende des röhrenförmigen Elements einen Mechanismus für das selektive Schließen der Öffnung zur Abdichtung der Kammer und Öffnen der Öffnung, um eine Überführung der medizinischen Vorrichtung durch die Öffnung zu ermöglichen, einschließt.

7. System nach Anspruch 5, das weiter ein Dichtungselement an dem zweiten Ende zur Abdichtung der Öffnung umfasst, wobei das Dichtungselement entfernbar ist, um eine Überführung der medizinischen Vorrichtung aus der Kammer durch die Öffnung zu ermöglichen.

8. System nach Anspruch 5, das weiter eine Membran an dem zweiten Ende umfasst, die eine flüssigkeitsdichte Dichtung zum Abdichten der Öffnung bereitstellt, wobei die Membran einen oder mehrere geschwächte Bereiche einschließt, die zum Reißen oder Brechen konfiguriert sind, wenn sie einer vorgegebenen Kraft unterzogen werden.

9. System nach Anspruch 5, das weiter eine Membran an dem zweiten Ende umfasst, die eine flüssigkeitsdichte Dichtung zum Abdichten der Öffnung bereitstellt, wobei die Membran zum Reißen oder Brechen konfiguriert ist, wenn die medizinische Vorrichtung in die Öffnung geleitet wird, um eine Überführung der medizinischen Vorrichtung aus der Kammer in die Abgabevorrichtung zu ermöglichen.

10. System nach einem vorstehenden Anspruch, wobei das röhrenförmige Element einen oder mehrere geschwächte Bereiche in einer Wand des röhrenförmigen Elements umfasst, die sich zumindest teilweise von dem ersten Ende in Richtung des zweiten Endes erstrecken, wobei das röhrenförmige Element ein oder mehrere Elemente zum Reißen des einen oder der mehreren geschwächten Bereiche zum Öffnen oder Trennen des röhrenförmigen Elements umfasst, um die Entfernung des röhrenförmigen Elements aus einer Einführanordnung, die in der Kammer gespült wurde, zu ermöglichen.

11. System nach Anspruch 10, wobei das eine oder die mehreren Elemente ein Ansatzstück an dem ersten Ende umfassen, das einen ersten und zweiten Teil einschließt, die getrennt werden können, um zu bewirken, dass der eine oder die mehreren geschwächten Bereiche von dem ersten Ende in Richtung des zweiten Endes reißen.

12. System nach einem vorstehenden Anspruch, das weiter eine erste Spülflüssigkeit innerhalb der Kammer umfasst, sodass eine medizinische Vorrichtung, die in die Kammer eingeführt wurde, der ersten Spülflüssigkeit ausgesetzt wird, bevor eine oder mehrere zusätzliche Spülflüssigkeiten in die Kammer eingeführt werden.

13. System nach einem vorstehenden Anspruch, das weiter eine Quelle für Kochsalzlösung umfasst, die mit dem ersten Anschluss zum Spülen des Lumens mit der Kochsalzlösung verbindbar ist, um die entgaste oder teilweise entgaste Lösung oder Perfluorkohlenwasserstofflösung aus einem oder beiden des Stentgrafts und der Kammer zu entfernen.

14. System nach einem vorstehenden Anspruch, das weiter eine Quelle für Spülgas umfasst, die mit einem des ersten und zweiten Anschlusses verbindbar ist, um einen Kreislauf zu erzeugen, um Luft oder andere Gase in einem oder beiden des Stentgrafts und der Kammer mit dem Spülgas vor dem Spülen mit der Perfluorkohlenwasserstofflösung auszutauschen, wobei das Spülgas Kohlendioxid oder biologisch inertes Gas ist.

15. Verfahren zur Entfernung von Gas aus einer medizinischen Vorrichtung, das Folgendes umfasst:
Bereitstellen eine Spülvorrichtung, die eine Kammer einschließt, die sich zwischen einem ersten und zweiten Ende davon erstreckt;
Einführen einer Einführanordnung, die ein Stentgraft trägt, das innerhalb einer Hülse gehalten wird, in die Kammer; und
Spülen der Kammer mit Perfluorkohlenwasserstofflösung;
wobei die Spülvorrichtung einen ersten und zweiten Anschluss an dem ersten bzw. zweiten Ende einschließt und wobei das Spülen der Kammer Folgendes umfasst:
Verbinden eine Quelle für Perfluorkohlenwasserstofflösung mit dem ersten Anschluss;
Verbinden eine Sammelquelle mit dem zweiten Anschluss;
Abgeben der Perfluorkohlenwasserstofflösung aus der Quelle durch den ersten Anschluss in die Kammer; und
Sammeln des gespülten Gases und überschüssiger Perfluorkohlenwasserstofflösung aus der Kammer durch den zweiten Anschluss in der Sammelquelle.

## Revendications

1. Système pour rincer un dispositif médical avant son introduction dans le corps d'un patient, comprenant :
un membre tubulaire allongé comprenant une première extrémité, une deuxième extrémité et une chambre dedans s'étendant entre la première et la deuxième extrémité ;
un ensemble d'introduction portant une endoprothèse reçue à l'intérieur de la chambre ;
un premier et un deuxième orifice espacés l'un de l'autre le long du membre tubulaire et communiquant avec la chambre ; et
une ou plusieurs sources de solution de perfluorocarbure pouvant être raccordées à l'un d'entre le premier et le deuxième orifice ou aux deux pour créer un circuit de rinçage délivrant la solution de perfluorocarbure dans le premier orifice, à travers la chambre, et hors du deuxième orifice pour retirer l'air ou d'autres gaz de l'une d'entre l'endoprothèse et la chambre ou des deux.

2. Système selon la revendication 1, dans lequel la chambre comprend une chambre centrale, et dans lequel le membre tubulaire comprend en outre une première et une deuxième chambre annulaire entourant partiellement au moins la chambre centrale, la première chambre annulaire communiquant avec le premier orifice et comprenant une première ouverture communiquant avec la chambre centrale à la première extrémité du membre tubulaire, la deuxième chambre annulaire communiquant avec le deuxième orifice et comprenant une deuxième ouverture communiquant avec la chambre centrale à la deuxième extrémité du membre tubulaire.

3. Système selon la revendication 1 ou la revendication 2, comprenant en outre un moyeu sur la première extrémité du membre tubulaire, le moyeu étant amovible de la première extrémité pour permettre de charger un dispositif médical dans la chambre pour rinçage, le moyeu pouvant être raccordé de nouveau à la première extrémité pour sceller la chambre après avoir chargé le dispositif médical dans la chambre.

4. Système selon la revendication 1 ou la revendication 2, comprenant en outre un moyeu sur la première extrémité du membre tubulaire comprenant un passage à travers celui-ci pour introduire un dispositif médical dans la chambre, le passage comprenant un ou plusieurs obturateurs pour permettre d'introduire le dispositif médical et fournir une garniture étanche au fluide.

5. Système selon l'une quelconque des revendications précédentes, dans lequel la deuxième extrémité du membre tubulaire comprend une ouverture pour transférer un dispositif médical à l'intérieur de la chambre après rinçage dans un dispositif de délivrance à travers l'ouverture.

6. Système selon la revendication 5, dans lequel la deuxième extrémité du membre tubulaire comprend un mécanisme pour sélectivement fermer l'ouverture pour sceller la chambre et ouvrir l'ouverture pour permettre le transfert du dispositif médical à travers l'ouverture.

7. Système selon la revendication 5, comprenant en outre un membre de scellement sur la deuxième extrémité pour sceller l'ouverture, le membre de scellement étant amovible pour permettre au dispositif médical d'être transféré de la chambre à travers l'ouverture.

8. Système selon la revendication 5, comprenant en outre une membrane sur la deuxième extrémité fournissant une garniture étanche au fluide scellant l'ouverture, la membrane comprenant une ou plusieurs régions affaiblies configurées pour se déchirer ou céder lorsque soumises à une force prédéterminée.

9. Système selon la revendication 5, comprenant en outre une membrane sur la deuxième extrémité fournissant une garniture étanche au fluide scellant l'ouverture, la membrane étant configurée pour se déchirer ou céder lorsque le dispositif médical est dirigé dans l'ouverture afin de permettre au dispositif médical d'être transféré de la chambre dans le dispositif de délivrance.

10. Système selon l'une quelconque des revendications précédentes, dans lequel le membre tubulaire comprend une ou plusieurs régions affaiblies dans une paroi du membre tubulaire s'étendant partiellement au moins de la première extrémité vers la deuxième extrémité, le membre tubulaire comprenant un ou plusieurs éléments pour déchirer l'une ou les plusieurs régions affaiblies pour ouvrir ou séparer le membre tubulaire afin de permettre de retirer le membre tubulaire d'autour d'un ensemble d'introduction rincé dans la chambre.

11. Système selon la revendication 10, dans lequel l'un ou les plusieurs éléments comprennent un moyeu à la première extrémité comprenant une première et une deuxième partie qui peuvent être séparées pour faire que l'une ou les plusieurs régions affaiblies se déchirent de la première extrémité vers la deuxième extrémité.

12. Système selon l'une quelconque des revendications précédentes, comprenant en outre un premier fluide de rinçage dans la chambre de telle sorte qu'un dispositif médical introduit dans la chambre est exposé au premier fluide de rinçage avant d'introduire un ou plusieurs fluides de rinçage additionnels dans la chambre.

13. Système selon l'une quelconque des revendications précédentes, comprenant en outre une source de solution saline pouvant être raccordée au premier orifice pour rincer la lumière avec la solution saline afin de retirer la solution dégazée ou partiellement dégazée ou la solution de perfluorocarbure de l'une d'entre l'endoprothèse et la chambre ou des deux.

14. Système selon l'une quelconque des revendications précédentes, comprenant en outre une source de gaz de rinçage pouvant être raccordée à l'un du premier et du deuxième orifice pour créer un circuit pour remplacer l'air ou d'autres gaz dans l'une d'entre l'endoprothèse et la chambre ou dans les deux avec le gaz de rinçage avant de rincer avec la solution de perfluorocarbure, où le gaz de rinçage est du dioxyde de carbone ou un gaz bio-inerte.

15. Procédé pour retirer du gaz d'un dispositif médical, comprenant :
fournir un dispositif de rinçage comprenant une chambre s'étendant entre une première et une deuxième extrémité de celui-ci ;
introduire un ensemble d'introduction portant une endoprothèse contrainte dans un fourreau dans la chambre ; et
rincer la chambre avec une solution de perfluorocarbure ;
dans lequel le dispositif de rinçage comprend un premier et un deuxième orifice à la première et à la deuxième extrémité, respectivement, et dans lequel rincer la chambre comprend :
coupler une source de solution de perfluorocarbure au premier orifice ;
coupler une source de recueil au deuxième orifice ;
délivrer la solution de perfluorocarbure de la source dans la chambre à travers le premier orifice ; et
recueillir le gaz rincé et la solution de perfluorocarbure excédentaire de la chambre dans la source de recueil à travers le deuxième orifice.
